(19) Europäisches Patentamt / European Patent Office / Office européen des brevets

(11) **EP 2 371 325 B1**

(12) **EUROPEAN PATENT SPECIFICATION**

(45) Date of publication and mention
of the grant of the patent:
**16.11.2016  Bulletin 2016/46**

(51) Int Cl.:
*A61F 7/02* *(2006.01)*          *A61F 7/00* *(2006.01)*

(21) Application number: **11151612.6**

(22) Date of filing: **21.01.2011**

(54) **Portable heating pad with improved control system**

Coussin chauffant portable avec un meilleur système de contrôle

Tragbares Heizkissen mit verbessertem Kontrollsystem

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **31.03.2010  IT MI20100546**

(43) Date of publication of application:
**05.10.2011  Bulletin 2011/40**

(73) Proprietor: **Tenacta Group S.p.A.**
**24052 Azzano S. Paolo (BG) (IT)**

(72) Inventors:
• **Morgandi, Arturo**
**24100, Bergamo (IT)**
• **Milani, Giancarlo**
**24030, Mozzo (BG) (IT)**

(74) Representative: **Pistolesi, Roberto et al**
**Dragotti & Associati Srl**
**Via Nino Bixio, 7**
**20129 Milano (IT)**

(56) References cited:
**EP-A1- 1 894 548      EP-A1- 1 977 709**
**GB-A- 2 160 965      US-A1- 2009 229 593**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

The present invention relates to a heating pad with electric heating and heat storage means, which makes use of the latent heat due to the phase change of a suitable material.

In the sector of heating pads (generally used for localized heating of parts of the body) apparatus which contain a heat storage mass and an electric circuit for heating this mass have been proposed. Once the storage mass has reached the desired temperature by means of the electric circuit, the apparatus may thus be disconnected from the electric power supply and used until it cools.

In order to attempt to obtain a temperature which is relatively constant during use, heating pads have been proposed where the heat storage mass is formed at least partially with a suitable material which undergoes a change in state at the desired temperature of use. In this way, by heating the heating pad to just above the temperature where a change in state occurs, it is possible to obtain a release of heat at constant temperature for the entire period of time required for the solidification state change. Apparatus of this type have been described, for example, in EP1894548, WO96/26694, WO00/24348, US2005/0021115 and GB2160965.

One problem of the known apparatus is due to the control of heating of the phase change material (abbreviated "PCM"). This is because excessive heating of said material results in a rapid deterioration thereof, in addition to a wastage of energy. It would therefore be preferable to avoid overtemperature situations (namely heating of the PCM to a temperature above its melting temperature). Moreover, in order to ensure optimum efficiency of the heating pad it would be preferable to ensure that all the PCM has changed phase into the liquefied state, so as to obtain the maximum amount of latent heat released during use. For this latter reason there is therefore the tendency, in any case, to ensure abundant heating, while at the same time attempting to keep the overtemperature within a safety range. For example, EP1894548 describes a system which measures the room temperature before starting heating of the heating pad and modifies the duration and/or the power of heating depending on the room temperature measured. Moreover, the heating temperature is monitored in order to interrupt heating in the event of an excessive overtemperature being detected.

In the known heating pads, correct heating and use are also complicated by the fact that these heating pads are usually made so as to be completely flexible.

In fact, since it is preferable to have a heating pad which is completely flexible so that it offers greater comfort during use, usually the phase change material is made in the form a thin pad which is inserted inside a flexible casing. When, however, the phase change material is converted from the solid state to the liquid or paste-like state it undergoes deformation which, after cooling, generally results in the heating pad assuming a deformed, and often twisted, rigid condition. As well as having an appearance which is unacceptable from an aesthetic point of view, giving the user the impression that the product is defective and/or of poor quality, the deformation may also result in there no longer being optimum contact between the phase change material and the electric heating circuit, with a reduction in the efficiency of the apparatus during subsequent use.

The use of a totally rigid containing structure furthermore results in difficulty of use, especially if it is required to have a heating pad with a relatively large surface area, which must be placed on a relatively broad zone of the body, such as the stomach, back or shoulders. The problem is increased by the need to have a containing structure which is strong and prevents excessive mechanical stressing of the electric heating part.

The main object of the present invention is to provide a heating pad which allows optimization of the heating stage and avoids unnecessary overtemperatures in the PCM.

A further object is to provide a heating pad which, during heating and use, maintains an optimum shape of the PCM and is strong, with suitable protection of the internal components, and which likewise is easy and comfortable to use.

In view of the above objects, the idea proposed according to the invention is to provide a heating pad with electric heating and heat storage means and a phase change material for maintaining the temperature, characterized in that it comprises an electronic system for controlling electric resistors for heating the phase change material, the electronic system comprising first means for measuring the temperature of the phase change material, second means for measuring the energy $Q_d$ supplied over time to the heating resistors, calculation means for calculating the quantity of energy Q necessary for heating the phase change material from the initial temperature measured by the first means to a desired final temperature, and comparison means which compare the quantity of energy Q calculated and the energy $Q_d$ supplied to the resistors in order to define an end condition of heating of the phase change material.

Still according to the principles of the invention, another idea proposed is to provide a method for electronically controlling the heating of a heating pad with electric heating and heat storage means and a phase change material for maintaining the temperature, comprising the steps of: detecting the initial temperature of the phase change material; calculating the quantity of energy Q necessary for heating the phase change material from the initial temperature measured to a desired final temperature; electrically powering the heating resistors and measuring the energy $Q_d$ supplied over time to the heating resistors; and comparing the quantity of energy Q calculated and the energy $Q_d$ supplied to the resistors in order to define an end condition of heating of the phase change material when $Q_d >= Q$.

In order to illustrate more clearly the innovative principles of the present invention and its advantages compared to the prior art, an example of embodiment applying these principles will be described below, with the aid of the accompanying drawings. In the drawings:

- Figure 1 shows a block diagram of a phase change heating pad according to the invention;
- Figure 2 shows an exploded, perspective, view of a possible advantageous embodiment of a heating pad according to the present invention;
- Figure 3 shows a partial, schematic, cross-sectional view of a detail of the heating pad according to Figure 2;
- Figure 4 shows an exploded schematic perspective view of a heating detail of the heating pad according to Figure 2;
- Figure 5 shows a perspective view of the heating pad according to Figure 2 assembled and inserted inside a protective bag.

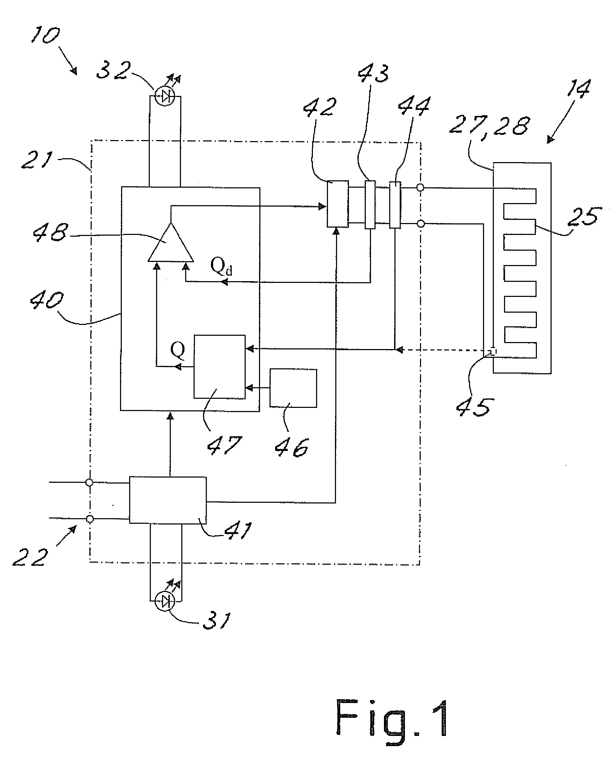

With reference to the figures, Figure 1 shows a block diagram of a heating pad - denoted generally by 10 - provided according to the invention.

The heating pad 10 comprises an electronic control system 21 which receives the electric power supply via a socket 22 and performs heating of a heating and heat storage assembly denoted generally by 14. The assembly 14 is formed by electric resistors 25 which are powered by the control system 21 and which heat a mass of PCM which is provided, for example, in the form of two layers or sheets 27, 28 between which the heating resistor is arranged.

The PCM is solid at room temperature and is chosen with a suitable phase transition temperature (melting temperature) which corresponds to the desired internal operating temperature of the heating pad.

Advantageously, the phase change temperature of the material is chosen so as to be between 55°C and 60°C and, in particular, equal to about 58°C. The quantity of PCM is chosen depending on the desired amount of heat to be stored.

The control system 21 comprises advantageously a control unit 40 which receives the power supply from a power supply unit 41 connected to the socket 22 and operates the heating resistors via a power unit 42. The control system comprises means 43 for measuring the energy $Q_d$ supplied to the resistors during the heating time. These means may comprise known sensors which can be easily imagined by the person skilled in the art. In particular, these means may comprise a known sensor 43 which measures the instantaneous power used by the resistors. The power measured is integrated over time in order to obtain the energy. In order to measure the power, the sensor 43 may be of the type which measures the current and the power supply voltage of the resistors and, based on said measurement, the sensor (or the unit 40) calculates the corresponding electric power. The unit 40 may also perform integration of the power over time.

The control system 21 also comprises means 44 for detecting the temperature of the PCM. These means may advantageously comprise a known sensor 44 which measures the impedance of the heating resistors. The electric resistors are in this case designed with an impedance which varies in a known manner depending on the temperature. It is thus possible to use the measured impedance value in order to calculate the temperature of the material with which the resistor comes into contact. Alternatively (or in addition) a known separate temperature sensor 45 (for example an NTC sensor) may also be used.

The control system also comprises an electronic memory 46 in which a number of characteristic parameters of the heating pad are stored, as will be explained below. The presence of the power supply may be signalled by a first indicator lamp 31, for example an LED, while heating may be signalled by a second indicator lamp 32, for example again an LED.

During heating of the heating pad 10, the control system operates using the regulating algorithm described below.

Firstly, when the apparatus is activated, the control system measures the temperature of the PCM (via the sensor 45 or the impedance measurement sensor 44) and calculates the energy Q required in order to heat the PCM from the initial temperature measured to a final desired temperature. This is performed using calculation means 47 of the control system which receive the temperature $t_i$ and, if necessary, a number of characteristic parameters of the heating pad contained in the memory 46.

The characteristic parameters may comprise or be dependent upon characteristics of the heating pad such as the transition temperature of the PCM used, the mass and the specific heat of the material to be heated.

For calculation of the energy Q, the calculation means may advantageously apply a function:

$$Q = f(t_i, t_f, t_t, m, c)$$

where:

$t_i$ = initial temperature measured;
$t_f$ = desired final temperature;
$t_t$ = transition temperature of the PCM used;
m = mass of material to be heated;
c = specific heat of the material to be heated.
"Material to be heated" is understood as meaning both the PCM and the materials surrounding it in the heating pad.

The temperature $t_i$ is measured as described above, while the remaining values of the formula are contained in the memory 46 as constant parameters which characterize the particular heating pad and which, advantageously, are defined a priori and entered into the memory when created. In general, the desired final temperature will be determined so as to coincide with or be slightly above the transition temperature of the PCM and such as not to produce an undesirable overtemperature of the PCM itself.

The control system may be advantageously formed by a unit 40 composed of a microprocessor system, known per se, suitably programmed to perform the various abovementioned functions via the software.

If a control system which has a calculation power insufficient to calculate directly the power Q is to be used, a table system, instead of direct calculation, may also be used. In other words, a table which contains a list of values Q (pre-calculated during design of the device) for each temperature value $t_i$ of interest is stored in the memory 46.

In this way, the calculation means 47 calculate the value Q simply by extracting it from the table, using the value $t_i$ as index in the table. If the value $t_i$ measured falls within two index values of the table, it is possible to use, for example, the value which is closest in the table or calculate an average of the two values of Q associated with these two values. The probability of it falling between the two index values $t_i$ will depend upon the interval chosen between the index values. This will also depend on the desired size of the table. For example, in order to create tables which are not large, it is possible to use a small number of values Q therefore having a relatively large interval between the index values $t_i$.

Other known calculation systems may obviously be used.

The control system, once the value Q has been calculated using the calculation means 47, activates the power supply of the heating resistors. The energy $Q_d$ supplied to the load is continuously monitored and compared, by means of comparison means 48, with the calculated energy Q. As mentioned above, the energy $Q_d$ may be obtained by monitoring continuously the electric power which is supplied to the electric load, represented by the heating resistors, and calculating $Q_d$ by integrating this power over time. When the energy $Q_d$ is supplied to the load it becomes equal to (or greater than) the energy Q calculated, the power supply to the load may be turned off and thus the product is ready for use.

Advantageously, during heating, the temperature of the PCM is continuously monitored in order to keep it below a predetermined threshold which prevents an unacceptable overtemperature of the PCM. If the temperature rises beyond the threshold, the control system switches off the heating elements for a predefined time period or until the temperature falls sufficiently below the threshold.

When the control system detects the end condition of the heating stage, it may signal this condition via the LEDs 32. The user is thus able to disconnect the heating pad from the power supply socket and use it until it cools, with the temperature which remains substantially constant until the end of the solidification state change.

Obviously, it is also possible to envisage maintaining the temperature reached, in the case where the heating pad is not used immediately, but is instead kept connected to the electric power supply. During this maintenance step, the heating pad control system will supply the electrical resistors with the right amount of power needed to maintain the final temperature reached.

The calculation means 47 and the comparator 48 may be advantageously formed via software using a special microprocessor suitably programmed to form the unit 40, as can be easily imagined by the person skilled in the art. Obviously, as can be easily imagined by the person skilled in the art, the control system may also be realized in the form of hardware.

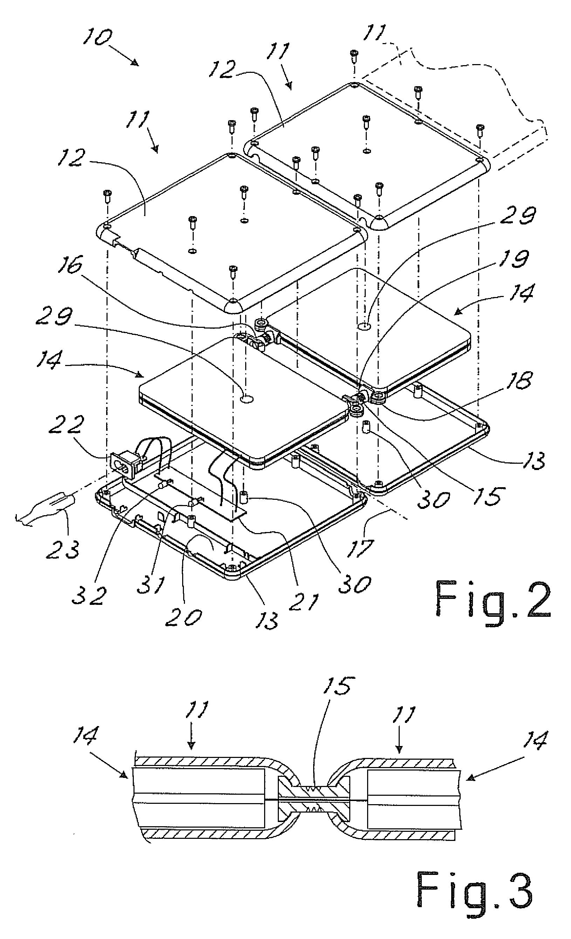

Figure 2 shows the exploded view of a possible advantageous embodiment of the heating pad 10 according to the invention.

Advantageously, as can be seen in Figure 2, according to this embodiment, the heating pad 10 comprises at least one pair of heating elements 11 which are hinged in sequence in their plane of extension so as to be articulated together and inclinable about a transverse axis 17. Each element 11 comprises two matching shells 12, 13 (advantageously made of rigid plastic) which are joined together to form a flat rectangular casing (for example with dimensions 12x12 cm), advantageously with rounded edges, which contains a respective heat module 14. Each module 14 may advantageously have a central hole 29 for centring on pins 30 projecting from the bottom of the containing shells 12, 13. The pins may also help keep the module centred in the thickness between the shells. The height of the internal space of the shells joined together is advantageously such as to house the heat modules with a minimum play at least in the direction perpendicular to their extension.

The hinging together of the two elements 11 is advantageously achieved along a side edge by means of a pair of (flexible or articulated) joints 15, 16 connected so as to form a bridge between facing sides of the two elements 10. Advantageously, the two joints are arranged close to two opposite edges of one side of the element 11.

The two joints 15, 16 allow advantageously the relative inclination of the two elements 11 about their facing sides (namely about the axis 17 transverse to the extension of the pair of interconnected elements), avoiding at the same time an uncontrolled relative rotation about an axis parallel to this extension, so that the facing side edges of the two elements 11 remain substantially parallel to each other.

In the embodiment shown, the joints are advantageously made of flexible plastic and have a generally elongate form with their axis directed perpendicular to the interconnected sides. For improved flexibility they also have suitable lateral incisions.

For a safe connection, each joint comprises advantageously two rings 18, 19, each one of which is close to one end of the joint situated inside the respective casing of the element 11, so as to be engaged with a suitable pin which forms the seat for a screw for closing the shells.

The first of the heating elements 11 comprises internally a compartment 20 which houses an electronic base-piece with the control system 21 connected to the heating modules. The control system 21 receives the electric power supply via the socket 22 which is flush-mounted on an edge of the element 11 and is intended to be connected to an electricity source by means of an electrical power supply cable provided with a mating connector 23.

As shown in Figure 3, the electrical connections between the heating modules pass axially through the joints 15, 16. Advantageously an outgoing conductor passes through one of the joints, while the return conductor passes through the other joint. The joints may be designed so that they can be opened or the conductors may be separable for axial introduction into the joints. Alternatively, the joint may also be moulded directly onto the respective conductor.

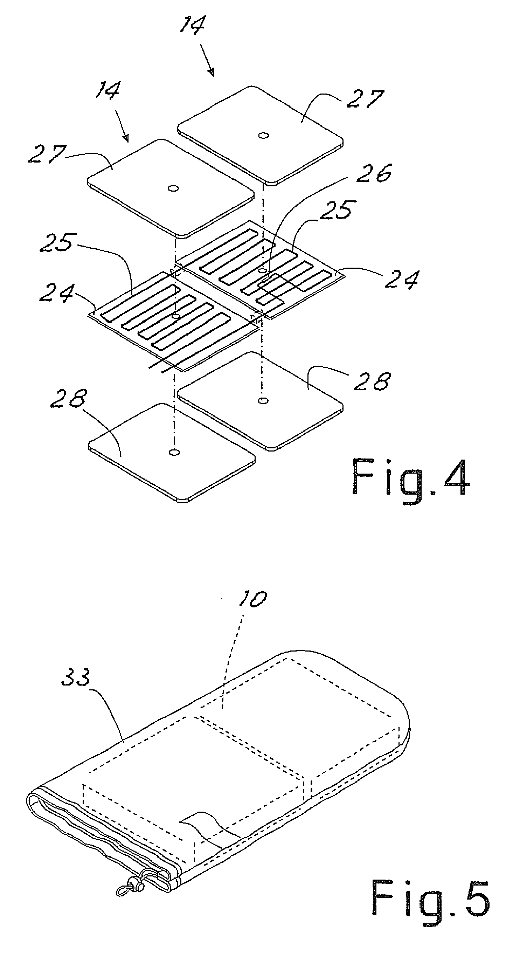

Figure 4 shows the structure of the heating modules 14 which has been found to be particularly advantageous. As can be seen in this figure, each module 14 comprises a central supporting base-piece 24 which is made of rigid and electrically insulating material (for example a suitable plastic which is able to withstand the heat produced by heating of the heating pad) and which contains the electric heating resistor 25 arranged in alternating loops so as to distribute the heat uniformly over the entire surface of the base-piece. The resistors of the various modules may be advantageously connected in series, as can be clearly seen in Figure 4. As described above, the electrical connections between one base-piece and the other base-piece pass through the joints 15, 16. In the case of temperature sensors separated by the electric resistors, the connections between these sensors and the control unit may also pass through the articulated joints.

Advantageously, at least one base-piece may be provided with a safety thermostat 26 which deactivates the resistors in the event of a predefined limit temperature being exceeded.

Two PCM layers 27, 28 are arranged on the two opposite faces of each base-piece.

As mentioned above, the volume of the layers will be chosen so as to produce a desired duration for release of latent heat during use of the heating pad. The PCM will be advantageously of the known type formed by a paraffin inserted in a polymer matrix or grid. This type of material has the advantage that, once its phase change temperature has been exceeded, it softens from the solid state, but the polymer structure prevents complete liquefaction of the entire layer.

During use of the heating pad, the pad is firstly connected to the electricity source in order to heat suitably the heat modules, as described above, using the control system 21 which will power the modules so as to reach and maintain, where necessary, the desired phase change temperature.

At the end of heating, the heating pad may be disconnected from the power supply and used until cooling occurs. Advantageously, when the heating pad is used, it may also be inserted inside a suitable bag 33 made of flexible material, such as fabric or the like, as shown in Figure 5.

At this point it is clear how the predefined objects have been achieved. The control system described enables a rapid and optimum heating action to be obtained, without wastage and without unacceptable overtemperatures of the PCM.

Moreover, owing to the advantageous structure according to the invention, it is possible to ensure cohesion between the layers of PCM and their correct connection to the walls of the casing and to the heating resistors. Moreover, owing to the system of hinging together the elements 11 (which are advantageously square in plan view), the heating pad may be easily adapted to the body. Relatively low manufacturing costs are also possible as a result of the simple structure.

Obviously, the above description of an embodiment applying the innovative principles of the present invention is provided by way of example of these innovative principles and must therefore not be regarded as limiting the scope of the rights claimed herein. The figures show only two elements 11 connected together, but it is clear that, when applying the principles of the present invention, it is possible to provide heating pads with any desired number of elements arranged in sequence (as shown schematically in broken lines in Figure 2). For this purpose, the intermediate elements will have the connection with articulated joints repeated on their opposite sides, as can be easily imagined by the person skilled in the art on the basis of the description provided above. It is thus possible, for example, to form a belt for the stomach or a "scarf" for the shoulders and the neck. Owing to the modular structure according to the invention, heating pads of different length with a different number of elements connected in series may also be produced at a minimum cost. If desired or preferred for particular needs, the resistors of the heating modules may also be connected in parallel, instead of in series, or a combination of the two.

**Claims**

1. Heating pad with electric heating and heat storage means and a phase change material (27, 28) for maintaining the temperature, **characterized in that** it comprises an electronic system (21) for controlling electric resistors (25) for heating the phase change material, the electronic system comprising first means (44) for measuring the temperature of the phase change material, second means (43) for measuring the energy $Q_d$ supplied over time to the heating resistors, calculation means (47) for calculating the quantity of energy Q necessary for heating the phase change material from the initial temperature measured by the first means (44) to a desired final temperature, and comparison means (48) which compare the quantity of energy Q calculated and the energy $Q_d$ supplied to the resistors in order to define an end condition of heating of the phase change material.

2. Heating pad according to Claim 1, **characterized in that** the heating resistors have an impedance variable depending on the temperature, and the first means for measuring the temperature comprise a sensor (44) for measuring the impedance of the heating resistors.

3. Heating pad according to Claim 1, **characterized in that** the second means for measuring the energy comprise sensors (43) for measuring the power used by the heating resistors.

4. Heating pad according to Claim 1, **characterized in that** it comprises an electronic memory (46) connected to the said calculation means (47) and containing characteristic parameters of the heating pad for calculating the quantity of energy Q as a function of the said initial temperature and said characteristic parameters.

5. Heating pad according to Claim 4, **characterized in that** the characteristic parameters comprise or are a function of the transition temperature of the phase change material, the mass and the specific heat of the material to be heated.

6. Heating pad according to Claim 1, **characterized in that** it comprises heating elements (11) which are connected together in sequence in a hingeable manner, each element (11) comprising in turn a rigid and flat outer casing (12, 13) which contains a heating module (14) comprising a central supporting base-piece (24) provided with electric heating resistors (25) and two layers (27, 28) arranged on opposite faces of the base-piece and made using the phase change heat storage material with a transition temperature which corresponds to the desired internal operating temperature of the heating pad.

7. Heating pad according to Claim 6, **characterized in that** the hinging system comprises two joints (15, 16) connected so as to form a bridge between two facing sides of two interconnected heating elements (11), and conductors for electrically connecting the electric heating resistors (25) pass through the joints (15, 16).

8. Heating pad according to Claim 6, **characterized in that** one of the heating elements comprises a socket (22) for connecting the heating pad to an electricity source and a compartment (20) containing the electronic control system (21) connected to the heating modules.

9. Heating pad according to Claim 1, **characterized in that** the phase change material comprises a paraffin inserted in a polymer matrix or grid.

10. Heating pad according to Claim 1, **characterized in that** the phase change temperature of the phase change material is between 55°C and 60°C and, in particular, is equal to about 58°C.

11. Method for electronically controlling the heating of a heating pad with electric heating and heat storage means and a phase change material (27, 28) for maintaining the temperature, comprising the steps:

   - detecting the initial temperature of the phase change material;
   - calculating the quantity of energy Q necessary for heating the phase change material from the initial temperature measured to a desired final temperature;
   - electrically powering the heating resistors and measuring the energy $Q_d$ supplied over time to the heating resistors;
   - comparing the quantity of energy Q calculated and the energy $Q_d$ supplied to the resistors in order to define an end condition of heating of the phase change material when $Q_d >= Q$.

12. Method according to Claim 11, wherein the electric heating resistors are designed with an impedance which is variable depending on the temperature, and detection of the initial temperature is performed by measuring this impedance.

13. Method according to Claim 11, wherein measurement of the energy $Q_d$ is performed by measuring the power used over time by the electric resistors and integrating it.

14. Method according to Claim 11, wherein calculation of the quantity of energy Q is performed as a function of the said initial temperature detected and characteristic parameters stored in the heating pad.

15. Method according to Claim 14, wherein the characteristic parameters comprise or are a function of the transition temperature of the phase change material, the mass and the specific heat of the material to be heated.

**Patentansprüche**

1. Heiz-Pad mit elektrischen Heiz- und Wärmespeicher-Mitteln und einem Phasenübergangs-Material (27, 28) zum Halten der Temperatur, **dadurch gekennzeichnet, dass** es umfasst, ein elektronisches System (21) zur Steuerung elektrischer Widerstände (25) zum Heizen des Phasenübergangs-Materials, worin das elektronische System umfasst, erste Mittel (44) zum Erfassen der Temperatur des Phasenübergangs-Materials, zweite Mittel (43) zum Erfassen der Energie $Q_d$, die über die Zeit zu den Heizwiderständen geliefert wurde, Berechnungsmittel (47), zum Berechnen der Menge der Energie Q, die erforderlich ist, um das Phasenübergangs-Material von der Anfangstemperatur, die von dem ersten Mittel (44) erfasst wurde, zu einer gewünschten End-Temperatur aufzuheizen, und Vergleichsmittel (48), welche die berechnete Energiemenge Q und die zu den Widerständen gelieferte Energiemenge $Q_d$ vergleichen, um einen Endzustand des Aufheizens des Phasenübergangs-Materials zu definieren.

2. Heiz-Pad nach Anspruch 1, **dadurch gekennzeichnet, dass** die Heizwiderstände eine von der Temperatur abhängige variable Impedanz aufweist, und dass die ersten Mittel zum Erfassen der Temperatur umfassen, einen Sensor (44) zum Erfassen der Impedanz der Heizwiderstände.

3. Heiz-Pad nach Anspruch 1, **dadurch gekennzeichnet, dass** die zweiten Mittel zur Erfassung der Energie umfassen, Sensoren (43) zur Erfassung des Stroms, der von den Heizwiderständen verbraucht wird.

4. Heiz-Pad nach Anspruch 1, **dadurch gekennzeichnet, dass** es umfasst, einen elektronischen Speicher (46), der mit den Berechnungsmitteln (47) verbunden ist, und charakteristische Parameter des Heiz-Pads zur Berechnung der Energiemenge Q als eine Funktion der Anfangs-Temperatur und der charakteristischen Parameter, enthält.

5. Heiz-Pad nach Anspruch 4, **dadurch gekennzeichnet, dass** die charakteristischen Parameter umfassen oder eine Funktion sind der Übergangs-Temperatur des Phasenübergangs-Materials, der Masse und der spezifischen Wärme des aufzuheizenden Materials.

6. Heiz-Pad nach Anspruch 1, **dadurch gekennzeichnet, dass** es umfasst, Heizelemente (11), die zusammen in Reihe in einer schwenkbaren Art und Weise verbunden sind, worin jedes Element (11) abwechselnd ein festes und flaches äußeres Gehäuse (12, 13) aufweist, das ein Heizmodul (14) enthält, das ein zentrales Träger-Basisstück (24) umfasst, das mit elektrischen Heizwiderständen (25) und zwei Schichten (27, 28) versehen ist, die auf abgewandten Seiten des Basis-Stück angeordnet und unter Verwendung des Phasenübergangs-Materials mit einer Übergangs-Temperatur hergestellt ist, welches der gewünschten internen Betriebstemperatur des Heiz-Pads entspricht.

7. Heiz-Pad nach Anspruch 6, **dadurch gekennzeichnet, dass** das Aufhängsystem zwei Verbindungen (15, 16) umfasst, die verbunden sind, um eine Brücke zwischen zwei gegenüberliegenden Seiten von zwei verbundenen Heizelementen (11) zu bilden, und Leiter, um die elektrischen Heiz-Widerstände (25), die durch die Verbindungen (15, 16) gelangen, zu verbinden.

8. Heiz-Pad nach Anspruch 6, **dadurch gekennzeichnet, dass** eines der Heizelemente eine Fassung (22) umfasst, um das Heiz-Pad mit einer Stromquelle zu verbinden, und einen Raum (20), der das elektronische Steuersystem (21) mit dem Heizmodulen verbindet.

9. Heiz-Pad nach Anspruch 1, **dadurch gekennzeichnet, dass** das Phasenübergangs-Material ein Paraffin umfasst, das in eine Polymermatrix oder ein Gitter inseriert ist.

10. Heiz-Pad nach Anspruch 1, **dadurch gekennzeichnet, dass** die PhasenübergangsTemperatur des Phasenübergangs-Materials zwischen 55 °C und 60 °C liegt, und insbesondere gleich etwa 58 °C ist.

11. Verfahren zur elektronischen Steuerung des Heizvorgangs eines Heiz-Pads mit einer elektrischen Heizung und Wärme-Speichermittel und einem Phasenübergangs-Material (27, 28) zum Halten der Temperatur, welches die Schritte umfasst:

   - Erfassen der Anfangstemperatur des Phasenübergangs-Materials;
   - Berechnen der Energiemenge Q, die zum Heizen des Phasenübergang-Materials von der erfassten Anfangstemperatur zu einer gewünschten Endtemperatur erforderlich ist;
   - Versorgen der Heizwiderstände mit Strom und Erfassen der über die Zeit zu den Heizwiderständen zugeführten Energie $Q_d$;
   - Vergleichen der berechneten Energiemenge Q und der zu den Widerständen zugeführten Energie $Q_d$, um einen Endzustand des Heizens des Phasenübergangs-Materials zu definieren, wenn $Q_d >= Q$.

12. Verfahren nach Anspruch 11, wobei die elektrischen Widerstände mit einer Impedanz ausgestattet sind, die in Abhängigkeit von der Temperatur variabel ist, und wobei das Erfassen der Anfangstemperatur durch Erfassen dieser Impedanz durchgeführt wird.

13. Verfahren nach Anspruch 11, wobei das Erfassen der Energie $Q_d$ durch Erfassen des Stroms, der über die Zeit von den elektrischen Widerständen verbraucht wird, und Integrieren, durchgeführt wird.

14. Verfahren nach Anspruch 11, wobei die Berechnung der Energiemenge Q als eine Funktion der erfassten Anfangstemperatur und charakteristischer Parameter, die in dem Heiz-Pad gespeichert sind, durchgeführt wird.

15. Verfahren nach Anspruch 14, wobei die charakteristischen Parameter umfassen oder eine Funktion sind der Übergangs-Temperatur des Phasenübergangs-Materials, der Masse und der spezifischen Wärme des zu heizenden Materials.

**Revendications**

1. Coussin chauffant à chauffage électrique, à moyen de stockage de la chaleur et à matériau de changement de phase (27, 28) destiné à maintenir la température, **caractérisé en ce qu'**il comprend un système électronique (21) destiné à commander des résistances électriques (25) afin de chauffer le matériau de changement de phase, le système électronique comprenant un premier moyen (44) de mesure de la température du matériau de changement de phase, un second moyen (43) de mesure de l'énergie $Q_d$ fournie au fil du temps aux résistances de chauffage, un moyen de calcul (47) destiné à calculer la quantité d'énergie Q nécessaire pour chauffer le matériau à changement de phase de la température initiale mesurée par le premier moyen (44) à une température finale souhaitée, et un moyen de comparaison (48) qui compare la quantité d'énergie Q calculée avec l'énergie $Q_d$ fournie aux résistances afin de définir une condition de fin de chauffage du matériau de changement de phase.

2. Coussin chauffant selon la revendication 1, **caractérisé en ce que** les résistances de chauffage possèdent une impédance variable selon la température, et le premier moyen de mesure de la température comprend un capteur (44) destiné à mesurer l'impédance des résistances de chauffage.

3. Coussin chauffant selon la revendication 1, **caractérisé en ce que** le second moyen de mesure de l'énergie comprend des capteurs (43) destinés à mesurer la puissance utilisée par les résistances de chauffage.

4. Coussin chauffant selon la revendication 1, **carac-** **térisé en ce qu'**il comprend une mémoire électronique (46) reliée audit moyen de calcul (47) et qui contient des paramètres caractéristiques du coussin chauffant afin de calculer la quantité d'énergie Q en fonction de ladite température initiale et desdits paramètres caractéristiques.

5. Coussin chauffant selon la revendication 4, **caractérisé en ce que** les paramètres caractéristiques comprennent ou dépendent de la température de transition du matériau de changement de phase, de la masse et de la chaleur spécifique du matériau à chauffer.

6. Coussin chauffant selon la revendication 1, **caractérisé en ce qu'**il comprend des éléments chauffants (11) qui sont reliés ensemble en séquence de manière articulée, chaque élément (11) comprenant à son tour un boîtier externe rigide et plat (12, 13) qui contient un module de chauffage (14) qui comprend une base de support centrale (24) munie de résistances de chauffage électriques (25) et de deux couches (27, 28) placées sur les faces opposées de la base et créées à l'aide du matériau de stockage de la chaleur à changement de phase avec une température de transition qui correspond à la température de fonctionnement interne souhaitée du coussin chauffant.

7. Coussin chauffant selon la revendication 6, **caractérisé en ce que** le système d'articulation comprend deux jonctions (15, 16) reliées de façon à former un pont entre deux côtés opposés de deux éléments de chauffage interconnectés (11), et des conducteurs destinés à relier électriquement les résistances de chauffage électriques (25) en passant par les jonctions (15, 16).

8. Coussin chauffant selon la revendication 6, **caractérisé en ce que** l'un des éléments chauffants comprend une fiche (22) destinée à relier le coussin chauffant à une source d'électricité et un compartiment (20) qui contient le système de commande électronique (21) relié aux modules de chauffage.

9. Coussin chauffant selon la revendication 1, **caractérisé en ce que** le matériau de changement de phase comprend une paraffine insérée dans une matrice ou un réseau polymérique.

10. Coussin chauffant selon la revendication 1, **caractérisé en ce que** la température de changement de phase du matériau de changement de phase est comprise entre 55°C et 60°C et, en particulier, est égale à environ 58°C.

11. Procédé de commande électronique du chauffage d'un coussin chauffant avec un chauffage électrique, un moyen de stockage de la chaleur et un matériau de changement de phase (27, 28) afin de maintenir la température, qui comprend les étapes qui consistent à :

    - détecter la température initiale du matériau de changement de phase ;
    - calculer la quantité d'énergie Q nécessaire pour chauffer le matériau à changement de phase de la température initiale mesurée à une température finale souhaitée ;
    - alimenter électriquement les résistances de chauffage et mesurer l'énergie $Q_d$ fournie au fil du temps aux résistances de chauffage ;
    - comparer la quantité d'énergie Q calculée avec l'énergie $Q_d$ fournie aux résistances afin de définir une condition de fin de chauffage du matériau de changement de phase.

**12.** Procédé selon la revendication 11, dans lequel les résistances de chauffage électriques sont conçues avec une impédance qui est variable en fonction de la température, et la détection de la température initiale est effectuée en mesurant cette impédance.

**13.** Procédé selon la revendication 11, dans lequel la mesure de l'énergie $Q_d$ est effectuée en mesurant la puissance utilisée au fil du temps par les résistances électriques et en l'intégrant.

**14.** Procédé selon la revendication 11, dans lequel le calcul de la quantité d'énergie Q est effectué en fonction de ladite température initiale détectée et des paramètres caractéristiques stockés dans le coussin chauffant.

**15.** Procédé selon la revendication 14, dans lequel les paramètres caractéristiques comprennent ou dépendent de la température de transition du matériau de changement de phase, de la masse et de la chaleur spécifique du matériau à chauffer.

Fig.1

Fig.2

Fig.3

Fig.4

Fig.5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1894548 A
- WO 9626694 A
- WO 0024348 A
- US 20050021115 A
- GB 2160965 A